# EUROPEAN PATENT APPLICATION

(11) **EP 4 792 820 A1**
(43) Date of publication of application: **19.08.2026**
(21) Application number: 24877204.8
(22) Date of filing: 09.10.2024
(51) Int. Cl.: A61K 35/19, A61P 17/02, C12N 5/078

(54) **METHOD FOR PRODUCING PLATELET-RICH PLASMA**

(30) Priority: 12.10.2023 JP 2023176866
(71) Applicant: AdipoSeeds, Inc., Tokyo 160-8582 (JP)
(72) Inventor: URUGA, Yukako, Tokyo 160-8582 (JP); MATSUBARA, Yumiko, Tokyo 160-8582 (JP)
(74) Representative: Script Intellectual Property LLP
(86) International application number: PCT/JP2024/036129
(87) International publication number: WO 2025/079609

(57) **Abstract**

It is an object of the present invention to provide a method for producing platelet-rich plasma in which the recovery rate of platelets from a blood sample is stably high, platelet-rich plasma produced by such a method, and the like. A method for producing platelet-rich plasma, including: a step A of subjecting a blood sample from a mammal to centrifugal separation at from 100 to 1000 G for from 1 to 30 minutes, and then collecting a supernatant; and a step B of subjecting the residual blood sample after the collection of the supernatant in the step A to centrifugal separation at from 100 to 1000 G for from 1 to 30 minutes, and then collecting a supernatant to obtain the platelet-rich plasma.

## Description

### Technical Field

The present invention relates to a method for producing platelet-rich plasma, and the like. In more detail, the present invention relates to a method for producing platelet-rich plasma in which the recovery rate of platelets from a blood sample is stably high, and the like.

### Background Art

Platelet-rich plasma (hereinafter, also referred to as "PRP") is plasma in which platelets are concentrated and which is prepared by centrifugal separation of blood. Such platelet-rich plasma contains various growth factors in abundance, and since these growth factors play effective roles in wound healing and tissue regeneration, the platelet-rich plasma is a promising material in the field of regenerative medicine (see, for example, Patent Document 1).

In addition, in a method for preparing a composition containing a blood-derived growth factor from mammalian blood, as a method for separating platelet-rich plasma from the blood, there is known a method including: subjecting the blood to a first centrifugal separation treatment at from 100 to 1000 G for from 1 to 30 minutes to collect an upper layer portion including plasma and a buffy coat; subjecting the upper layer portion to a second centrifugal separation treatment at from 1000 to 2500 G for from 1 to 30 minutes to pelletize platelets and buffy coat components; and removing a supernatant and suspending the platelets and the buffy coat components to obtain the platelet-rich plasma (Patent Document 2).

### Prior Art Documents

### Patent Documents

Patent Document 1: Japanese unexamined Patent Application Publication No. 2009-195739
Patent Document 2: Japanese Patent No. 7175055

### Summary of the Invention

### Object to be Solved by the Invention

The recovery rate of platelets varies relatively greatly depending on individual differences among mammals as sources from which blood samples are collected, and in conventional methods for producing platelet-rich plasma, the recovery rate of the platelets from the blood samples may be low depending on the individual mammals.

It is an object of the present invention to provide a method for producing platelet-rich plasma in which the recovery rate of platelets from a blood sample is stably high, platelet-rich plasma produced by such a method, and the like.

### Means to Solve the Object

In order to solve the above object, the present inventors have conducted extensive studies on various methods, and have found that the above object can be solved by subjecting a blood sample from a mammal to centrifugal separation at from 100 to 1000 G for from 1 to 30 minutes, then collecting a supernatant, subjecting the residual blood sample after the collection of the supernatant to centrifugal separation at from 100 to 1000 G for from 1 to 30 minutes, and then collecting a supernatant, thereby completing the present invention.

In addition, the present inventors have found that, although conventional clinical PRP preparation kits currently commercially available from various companies cannot sufficiently suppress contamination with red blood cells and white blood cells into PRP, the method for producing PRP according to the present invention can effectively suppress the contamination with the red blood cells and the white blood cells into the PRP, thereby completing the present invention.

In other words, according to the present invention, the following inventions and the like are provided.
(1) A method for producing platelet-rich plasma, including:
   a step A of subjecting a blood sample from a mammal to centrifugal separation under centrifugal separation conditions of from 100 to 1000 G for from 1 to 30 minutes, and then collecting a supernatant; and
   a step B of subjecting the residual blood sample after the collection of the supernatant in the step A to centrifugal separation under centrifugal separation conditions of from 100 to 1000 G for from 1 to 30 minutes, and then collecting a supernatant to obtain the platelet-rich plasma.
(2) The method according to (1) described above, wherein the centrifugal separation conditions in the step A are from 100 to 300 G for from 5 to 15 minutes, and the centrifugal separation conditions in the step B are from 100 to 300 G for from 5 to 15 minutes.
(3) The method according to (1) or (2) described above, wherein the blood sample from the mammal to be subjected to the centrifugal separation in the step A is a blood sample stored at from 0 to 35°C for from 1 to 120 hours after the collection from the mammal.
(4) The method according to any one of (1) to (3) described above, wherein the blood sample from the mammal to be subjected to the centrifugal separation in the step A is a blood sample to which an anticoagulant agent has been added after the collection from the mammal.
(5) Platelet-rich plasma produced by the method according to any one of (1) to (4) described above.

### Effect of the Invention

According to the present invention, it is possible to provide a method for producing platelet-rich plasma in which the recovery rate of platelets from a blood sample is stably high, platelet-rich plasma produced by the method, and the like.

### Brief Description of Drawing

[Figure 1]

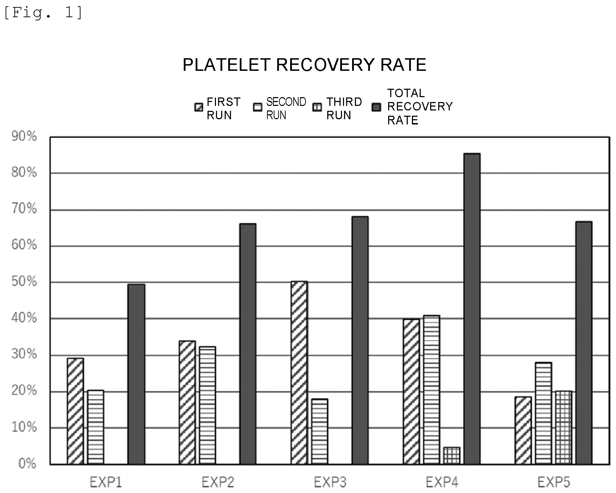

Figure 1 is a diagram illustrating the recovery rate (%) of platelets in a supernatant after centrifugal separation in the production of platelet-rich plasma in Examples. The recovery rate (%) of the platelets is expressed as a percentage (%) relative to the number of platelets in whole blood of a test subject. On the horizontal axis, "EXP1" to "EXP5" respectively represent five healthy volunteers as test subjects. In the results for each test subject, the leftmost result represents the recovery rate (%) of the platelets in the supernatant after the first centrifugal separation, and the second result from the left represents the recovery rate (%) of the platelets in the supernatant after the second centrifugal separation. For EXP4 and EXP5, the third result from the left represents the recovery rate (%) of the platelets in the supernatant after the third centrifugal separation. In the results for "EXP1" to "EXP3", the rightmost result represents the sum of the first and the second recovery rates (total recovery rate (%)), and in the results for "EXP4" and "EXP5", the rightmost result represents the sum of the first to third recovery rates (total recovery rate (%)).

### Mode of Carrying Out the Invention

The present invention includes:
[1] a method for producing platelet-rich plasma (hereinafter, also referred to as "the method for producing according to the present invention"), including:
   a step A of subjecting a blood sample from a mammal to centrifugal separation under centrifugal separation conditions of from 100 to 1000 G for from 1 to 30 minutes, and then collecting a supernatant; and
   a step B of subjecting the residual blood sample after the collection of the supernatant in the step A to centrifugal separation at from 100 to 1000 G for from 1 to 30 minutes, and then collecting a supernatant to obtain the platelet-rich plasma; and
[2] platelet-rich plasma produced by the method for producing according to the present invention (hereinafter, also referred to as "the platelet-rich plasma of the present invention");
   and the like.

### <Method for Producing According to the Present Invention>

The method for producing according to the present invention is not particularly limited, as long as it is a method for producing platelet-rich plasma including:
a step A of subjecting a blood sample from a mammal to centrifugal separation under centrifugal separation conditions of from 100 to 1000 G for from 1 to 30 minutes, and then collecting a supernatant; and
a step B of subjecting the residual blood sample after the collection of the supernatant in the step A to centrifugal separation under centrifugal separation conditions of from 100 to 1000 G for from 1 to 30 minutes, and then collecting a supernatant to obtain the platelet-rich plasma.

### (Step A)

Step A is not particularly limited as long as it is a step of subjecting the blood sample from the mammal to centrifugal separation under centrifugal separation conditions of from 100 to 1000 G for from 1 to 30 minutes, and then collecting a supernatant.

Examples of the "mammal" described above include a human and a non-human mammal, for example, and a human is preferred. In addition, examples of the non-human mammal include, a mouse, a rat, a guinea pig, a dog, a cat, a monkey, a rabbit, a sheep, a horse, and a pig, for example.

The "blood sample" described above is not particularly limited as long as it is a blood sample containing platelets, and preferred examples thereof include whole blood. A blood sample may be collected from a mammal by a conventional method.

The "blood sample" to be subjected to centrifugal separation in the step A is preferably a blood sample to which an anticoagulant agent has been added after the collection from the mammal. In addition, the anticoagulant agent may be added later to the collected blood sample; however, it is desirable to collect the blood sample into a container such as a blood collection tube or a blood collection bag containing an anticoagulant agent in advance. Examples of the "anticoagulant agent" described above include a chelating agent that suppresses blood coagulation by coordinating calcium ions that cause blood coagulation, and an antithrombin agent including heparin that suppresses thrombin activity, which causes the blood coagulation. Among these, the chelating agent is preferred. Examples of the anticoagulant agent that is a chelating agent include one or more selected from the group consisting of citric acid, EDTA (ethylenediaminetetraacetic acid), DTPA (diethylenetriaminepentaacetic acid), DCTA (1,2-diaminocyclohexanetetraacetic acid), EGTA (ethylene glycol bis(2-aminoethyl ether)tetraacetic acid), oxalic acid, or salts thereof. Among these, citric acid or a salt thereof is preferred. As the salt, an alkali metal salt is preferred, and a sodium salt is more preferred.

The anticoagulant agent may be added as a solid; however, it may also be added as a solution (anticoagulant solution) prepared by dissolving the anticoagulant agent in a solvent such as purified water (preferably a physiologically acceptable solvent). Specific examples of the aqueous solution containing an anticoagulant agent include an ACD-A solution (an aqueous solution containing sodium citrate hydrate, citric acid hydrate, and glucose), a CPD solution (an aqueous solution containing sodium citrate hydrate, citric acid hydrate, glucose, and sodium dihydrogen phosphate hydrate), and a CPDA-1 solution (an aqueous solution containing sodium citrate hydrate, citric acid hydrate, glucose, sodium dihydrogen phosphate hydrate, and adenine). Among these, the ACD-A solution is preferred.

As the anticoagulant agent, only one type may be used, or two or more types may be used in combination. Commercially available anticoagulant agents and anticoagulant solutions may be used.

The concentration of the anticoagulant agent in the blood sample to which the anticoagulant agent (or the anticoagulant solution) has been added is not particularly limited as long as the effects of the present invention can be obtained. However, for example, when the anticoagulant agent is citric acid or a salt thereof, the citric acid equivalent concentration in the blood sample to which the anticoagulant agent (or the anticoagulant solution) has been added is, for example, from 0.2 to 3 w/v%, preferably from 0.5 to 2 w/v%. Similarly, when the anticoagulant agent is EDTA or a salt thereof, the EDTA equivalent concentration is, for example, from 0.2 to 3 w/v%, preferably from 0.5 to 2 w/v%. When the anticoagulant agent is DTPA or a salt thereof, the DTPA equivalent concentration is, for example, from 0.2 to 3 w/v%, preferably from 0.5 to 2 w/v%. When the anticoagulant agent is DCTA or a salt thereof, the DCTA equivalent concentration is, for example, from 0.2 to 3 w/v%, preferably from 0.5 to 2 w/v%. When the anticoagulant is EGTA or a salt thereof, the EGTA equivalent concentration is, for example, from 0.2 to 3 w/v%, preferably from 0.5 to 2 w/v%. When the anticoagulant agent is oxalic acid or a salt thereof, the oxalic acid equivalent concentration is, for example, from 0.2 to 3 w/v%, preferably from 0.5 to 2 w/v%. In addition, when two or more anticoagulant agents are used in combination, the total concentration thereof may be, for example, from 0.2 to 3 w/v%, preferably from 0.5 to 2 w/v%.

The "blood sample" to be subjected to the centrifugal separation in the step A need not have been stored for, for example, 1 hour or more after being collected from the mammal. However, when the storage time is long, the recovery rate of the platelets tends to decrease. From the viewpoint of obtaining more benefits of the present invention, the "blood sample" to be subjected to the centrifugal separation in the step A is preferably a blood sample stored for, for example, 1 hour or more after the collection from the mammal. The lower limit of the storage time for the blood sample stored for one hour or more may be preferably, for example, 3 hours or more, 8 hours or more, 12 hours or more, 18 hours or more, 24 hours or more, 36 hours or more, 48 hours or more, 60 hours or more, 72 hours or more, 84 hours or more, 96 hours or more, 108 hours or more, or 120 hours or more, and the upper limit of the storage time may be, for example, 14 days or less, 12 days or less, 10 days or less, 8 days or less, or 6 days or less. These lower limits and upper limits may be combined arbitrarily. In addition, the temperature at which the blood sample is stored is not particularly limited, and examples thereof include, for example, from 0 to 35°C, from 0 to 30°C, from 0 to 25°C, from 0 to 20°C, from 0 to 15°C, from 0 to 10°C, and from 0 to 8°C.

The centrifugal separation conditions in the step A are not particularly limited as long as they are centrifugal separation conditions of from 100 to 1000 G for from 1 to 30 minutes. The centrifugal force under these conditions is not particularly limited as long as it is from 100 to 1000 G, and may be preferably from 100 to 900 G, from 100 to 800 G, from 100 to 700 G, from 100 to 600 G, from 100 to 500 G, from 100 to 400 G, from 100 to 300 G, from 100 to 250 G, from 150 to 900 G, from 150 to 800 G, from 150 to 700 G, from 150 to 600 G, from 150 to 500 G, from 150 to 400 G, from 150 to 300 G, or from 150 to 250 G, more preferably from 100 to 500 G, from 100 to 400 G, from 100 to 300 G, from 100 to 250 G, from 150 to 500 G, from 150 to 400 G, from 150 to 300 G, or from 150 to 250 G, and still more preferably from 100 to 300 G, from 100 to 250 G, from 150 to 300 G, or from 150 to 250 G. The centrifugation time under these conditions is not particularly limited as long as it is from 1 to 30 minutes, and may be preferably from 2 to 25 minutes, from 3 to 25 minutes, from 4 to 25 minutes, from 5 to 25 minutes, from 2 to 20 minutes, from 3 to 20 minutes, from 4 to 20 minutes, or from 5 to 20 minutes, more preferably from 5 to 18 minutes, from 6 to 18 minutes, from 7 to 18 minutes, from 5 to 15 minutes, from 6 to 15 minutes, from 7 to 15 minutes, from 5 to 13 minutes, from 6 to 13 minutes, or from 7 to 13 minutes.

The centrifugal separation of the blood sample may be performed using a commercially available centrifugal separator or the like.

The method for collecting the supernatant after the centrifugal separation in the step A is not particularly limited, and examples thereof include a method for collecting all plasma fractions (a platelet-rich plasma fraction and a platelet-poor plasma fraction), or a method for collecting a platelet-rich plasma fraction.

When collecting the supernatant, any volume may be collected so as to avoid including a fraction containing red blood cells (for example, visually) as much as possible. In detail, for example, based on the total volume of the liquid after the centrifugal separation in the step A, the volume ratio of the supernatant to be collected may be preferably from 1:0.1 to 1:0.5, more preferably from 1:0.2 to 1:0.5, and still more preferably from 1:0.3 to 1:0.5.

### (Step B)

The step B is not particularly limited as long as it is a step of subjecting the residual blood sample after the collection of the supernatant in the step A to centrifugal separation under centrifugal separation conditions of from 100 to 1000 G for from 1 to 30 minutes, and then collecting the supernatant to obtain the platelet-rich plasma. It should be noted that the "residual blood sample" to be subjected to the centrifugal separation in the step B may be the blood sample itself remaining after the collection of the supernatant in the step A (that is, the residual blood sample), or may be a residual blood sample obtained by carrying out some additional treatment on the residual blood sample, as long as the effects of the present invention can be obtained. It should be noted that the recovery rate of the platelets varies relatively greatly depending on individual differences among mammals as sources from which the blood samples are collected. In the method for producing according to the present invention, by involving the step B in addition to the step A, it is possible to reduce the influence of individual differences among mammals as the sources from which the blood samples are collected, and to stably maintain a high recovery rate of the platelets.

The centrifugal separation conditions in the step B are not particularly limited as long as they are centrifugal separation conditions of from 100 to 1000 G for from 1 to 30 minutes. The centrifugal force under these conditions is not particularly limited as long as it is from 100 to 1000 G, and may be preferably from 100 to 900 G, from 100 to 800 G, from 100 to 700 G, from 100 to 600 G, from 100 to 500 G, from 100 to 400 G, from 100 to 300 G, from 100 to 250 G, from 150 to 900 G, from 150 to 800 G, from 150 to 700 G, from 150 to 600 G, from 150 to 500 G, from 150 to 400 G, from 150 to 300 G, or from 150 to 250 G, more preferably from 100 to 500 G, from 100 to 400 G, from 100 to 300 G, from 100 to 250 G, from 150 to 500 G, from 150 to 400 G, from 150 to 300 G, or from 150 to 250 G, and still more preferably from 100 to 300 G, from 100 to 250 G, from 150 to 300 G, or from 150 to 250 G. The centrifugation time under these conditions is not particularly limited as long as it is from 1 to 30 minutes, and may be preferably from 2 to 25 minutes, from 3 to 25 minutes, from 4 to 25 minutes, from 5 to 25 minutes, from 2 to 20 minutes, from 3 to 20 minutes, from 4 to 20 minutes, or from 5 to 20 minutes, and more preferably from 5 to 18 minutes, from 6 to 18 minutes, from 7 to 18 minutes, from 5 to 15 minutes, from 6 to 15 minutes, from 7 to 15 minutes, from 5 to 13 minutes, from 6 to 13 minutes, or from 7 to 13 minutes.

It should be noted that the centrifugal separation conditions in the step A and the centrifugal separation conditions in the step B may either be the same or different.

The method for collecting the supernatant after the centrifugal separation in the step B is not particularly limited, and preferred examples thereof include a method for collecting the supernatant from a portion containing a small amount of a white blood cell component and/or a red blood cell component. Such supernatant contains platelets at a relatively high concentration.

When collecting the supernatant, any volume may be collected so as to avoid including a fraction containing red blood cells (for example, visually) as much as possible. In detail, for example, based on the total volume of the liquid after the centrifugal separation in the step B, the volume ratio of the supernatant to be collected may be preferably from 1:0.1 to 1:0.9, more preferably from 1:0.1 to 1:0.6, and still more preferably from 1:0.1 to 1:0.4.

### (Optional Steps)

The method for producing according to the present invention may include only the step A and the step B, but may further include other optional steps. Examples of such optional steps include:
a step C of subjecting the residual blood sample after the collection of the supernatant in the step B to centrifugal separation under centrifugal separation conditions of from 100 to 1000 G for from 1 to 30 minutes and then collecting the supernatant to obtain platelet-rich plasma. It should be noted that the recovery rate of platelets varies relatively greatly depending on individual differences among mammals as sources from which the blood samples are collected. The step C may be preferably carried out from the viewpoint of further improving the recovery rate of the platelets when the recovery rate of the platelets is not particularly high even after carrying out the step A and the step B. It should be noted that the centrifugal separation conditions in the step C may either be the same as or different from the centrifugal separation conditions in the step A and the step B. Further, in the step C described above, the step C may be further repeated by using a "residual blood sample after the collection of the supernatant" in the step C instead of the "residual blood sample after the collection of the supernatant in the step B." The total number of centrifugal separation runs in the case of carrying out from the step A to the step C, or further repeating the step C, is not particularly limited, and may be, for example, from 3 to 6 times or from 3 to 4 times. When the step C is repeated, the centrifugal separation conditions may either be the same or different.

Other optional steps other than the step C described above include:
a step D of removing blood cells other than the platelets from the platelet-rich plasma obtained in the step B or the step C (or, when the step C is repeated, the step C carried out last).

Examples of a method for removing the blood cells other than the platelets from the platelet-rich plasma include filtering, for example. It should be noted that, since the method for producing according to the present invention enables efficient preparation of the platelet-rich plasma with a low level of contamination with blood cells such as white blood cells, the treatment in the step D described above (for example, filtering) may not be carried out.

Examples of other optional steps other than the step C and the step D described above include:
a step E of calcium-stimulating the platelets in the platelet-rich plasma obtained in the step B or the step C (or, when the step C is repeated, the step C carried out last) or in the step D.

Calcium stimulation of the platelets according to the present invention causes the platelets to secrete a greater amount of cytokines, thereby providing platelet-rich plasma with further improved anti-inflammatory effects and the like. The method for calcium stimulation is not particularly limited, and examples thereof include a method for incubating the platelets in the platelet-rich plasma of the present invention in a solution having a calcium concentration of, for example, from 0.01 to 100 mM, preferably from 0.5 to 50 mM, more preferably from 5 to 20 mM. The incubation time is not particularly limited, and may be, for example, from 0.1 minutes to 3 hours, preferably 10 to 20 minutes.

The calcium-containing compound used for the calcium stimulation is not particularly limited, and examples thereof include a calcium salt such as calcium chloride.

In addition, the timing of the calcium stimulation is not particularly limited, but examples thereof include a time prior to administration of the platelet-rich plasma of the present invention to a mammal (preferably a human) or the like having a disease, for example, within 5 hours, within 3 hours, within 1 hour, or within 30 minutes prior to the time of administration.

Examples of other optional steps other than the step C, the step D, and the step E described above include:
a step F of freezing the platelet-rich plasma obtained in the step B or the step C (or, when the step C is repeated, the step C carried out last), or in the step D or in the step E. The freezing may be a freezing treatment that is not freeze drying, or may be a freeze drying treatment. Frozen platelet-rich plasma is preferable in that it can be stored for a longer period.

When the method for producing according to the present invention includes the optional steps, the method for producing according to the present invention may include, as optional steps, one or more (two, three, or four) selected from the group consisting of the step C, the step D, the step E, and the step F, in addition to the essential steps A and B.

### (Total Recovery Rate of Platelets)

The total recovery rate (%) of the platelets in the method for producing according to the present invention is not particularly limited, and examples of the lower limit thereof include 40% or more, preferably 45% or more, 50% or more, 55% or more, and 60% or more, and examples of the upper limit thereof include 100% or less, 95% or less, 90% or less, 85% or less, and 80% or less. These lower limits and upper limits can be arbitrarily combined. As used herein, the term "total recovery rate (%) of platelets" means the ratio (%) of the total number of platelets recovered by the method for producing according to the present invention relative to the total number of platelets in the blood sample in the step A.

### <Platelet-Rich Plasma of the Present Invention>

The platelet-rich plasma of the present invention is not particularly limited as long as it is platelet-rich plasma produced by the method for producing according to the present invention.

It has been pointed out that contamination of a platelet-rich plasma (PRP) preparation with red blood cells may lead to adverse effects such as microcirculatory dysfunction, vascular injury, tissue damage, and degenerative joint damage (Biomedicines 2023, 11, 2425. https://doi.org/10.3390/biomedicines11092425). The number of red blood cells in the platelet-rich plasma of the present invention is not particularly limited, and examples thereof include 10,000/µL or less and 1,000/µL or less, preferably 500/µL or less, 300/µL or less, 200/µL or less, 100/µL or less, and 50/µL or less. In addition, the number of white blood cells in the platelet-rich plasma of the present invention is not particularly limited, and examples thereof include 500/µL or less, preferably 300/µL or less, 200/µL or less, and 100/µL or less.

The concentration of vascular endothelial growth factor (VEGF) in the platelet-rich plasma of the present invention is not particularly limited, and may preferably be 100 pg/mL, more preferably 300 pg/mL or more, still more preferably 700 pg/mL or more, and even more preferably 1,000 pg/mL or more. The upper limit varies depending on individual differences among mammals from which the blood samples are collected, and examples thereof include 30,000 pg/mL.

The platelet-rich plasma of the present invention may be contained in a container. The material and the shape of the container are not particularly limited, and examples thereof include a cryotube, a cryovial, a freezing bag, and an infusion bag, for example.

The platelet-rich plasma of the present invention can be used for any purpose, such as blood transfusion and regenerative medicine.

The present invention will be described in more detail based on the examples below. However, the present invention is not limited to these examples.

### Example 1

### Test 1. [Confirmation of Recovery Rate of Platelets by the Method for Producing According to the Present Invention]

In order to confirm the recovery rate of platelets by the method for producing according to the present invention, the following test was conducted.

Three milliliters each of a citrate dextrose solution (ACD solution) as an anticoagulant agent was dispensed into five 20-mL syringes. Using the above-described syringes, approximately 20 mL of blood was collected from each of five healthy volunteers ("EXP1" to "EXP5"), and the whole blood was dispensed into polypropylene tubes, respectively. The platelet count in each whole blood sample was measured.

The five polypropylene tubes containing the whole blood as described above were set in a centrifugal separator, and were subjected to centrifugal separation at 200 G for 10 minutes, and then the plasma as the supernatant (about 5 mL: based on the total volume of the liquid after the centrifugal separation, the collected volume ratio was 1:0.4) was collected. The platelet count in the collected supernatant was measured using a blood cell analyzer, and the recovery rate (%) relative to the platelet count in the whole blood was calculated. The results are shown in Figure 1 as the results of the "first" centrifugal separation.

As can be seen from Figure 1, the recovery rate of the platelets by the first centrifugal separation was from 20% to 50%.

The remaining blood (residual blood) samples after the collection of the supernatant in the first centrifugal separation as described above were then subjected to a second centrifugal separation at 200 G for 10 minutes, and then the plasma as the supernatant (about 3 mL: based on the total volume of the liquid after the second centrifugal separation, the collected volume ratio was 1:0.15) was collected. The platelet count in the collected supernatant was measured using a blood cell analyzer, and the recovery rate (%) relative to the platelet count in the whole blood was calculated. The results are shown in Figure 1 as the results of the "second" centrifugal separation.

As can be seen from Figure 1, the recovery rate of the platelets by the second centrifugal separation was from 15% to 40%. Further, it is shown that, when the recovery rate of the first centrifugal separation and the recovery rate of the second centrifugal separation are summed, a high recovery rate of approximately from 50% to 80% is achieved.

For EXP4 and EXP5, the remaining blood (residual blood) samples after the collection of the supernatant in the second centrifugal separation were then subjected to a third centrifugal separation at 200 G for 10 minutes, and then the plasma as the supernatant (about 2 mL: based on the total volume of the liquid after the third centrifugal separation, the collected volume ratio was 1:0.13) was collected. The platelet count in the collected supernatant was measured using a blood cell analyzer, and the recovery rate (%) relative to the platelet count in the whole blood was calculated. The results are shown in Figure 1 as the results of the "third" centrifugal separation.

As can be seen from Figure 1, the recovery rate of the platelets by the third centrifugal separation was from 4% to 20%. It shows that, when the total recovery rate of the first and second centrifugal separations is relatively low, platelets can be recovered in a somewhat larger amount by the third centrifugal separation.

In Figure 1, the sum of the recovery rates of the first and second centrifugal separations for EXP1 to EXP3 and the sum of the recovery rates of the first, second, and third centrifugal separations for EXP4 and EXP5 are shown as the total recovery rate. From these results, it is shown that according to the method for producing platelet-rich plasma of the present invention, platelets can be recovered from a blood sample at a high recovery rate.

### Test 2. [Confirmation of Red Blood Cell Count and White Blood Cell Count in Platelets Obtained by the Method for Producing According to the Present Invention]

It has been pointed out that contamination of a platelet-rich plasma (PRP) preparation with red blood cells may lead to adverse effects such as microcirculatory dysfunction, vascular injury, tissue damage, and degenerative joint damage (Biomedicines 2023, 11, 2425. https://doi.org/10.3390/biomedicines11092425). Accordingly, in order to confirm the red blood cell count and the white blood cell count in the platelet-rich plasma produced by the method for producing according to the present invention, the following test was conducted.

Blood was collected from 15 healthy volunteers, and in accordance with the method described in Test 1 described above, centrifugal separation was performed twice. Then, for each of the PRPs for which a recovery rate of platelet was 75% or more, the red blood cell count and the white blood cell count were measured using an automatic blood cell counter CBC LC-661 (manufactured by Fukuda Denshi). The results are shown in Table 1.

**[Table 1]**

| Red blood cell count (measured at n = 15) (unit: 1 × 10⁶ cells/µL) | White blood cell count (measured at n = 15) (unit: 1 × 10³ cells/µL) |
|---|---|
| 0.0 (results at n = 15) | 0.0 (results at n = 12) |
| | 0.2 (results at n = 3) |

From Table 1, it is shown that the PRP produced by the method for producing according to the present invention contains extremely small numbers of contaminating red blood cells and white blood cells. It indicates that the method for producing according to the present invention is remarkably excellent as a method for producing the PRP even in an embodiment in which the centrifugal separation is carried out twice (that is, an embodiment including the step A and the step B but not including the step C).

### Industrial Applicability

According to the present invention, it is possible to provide a method for producing platelet-rich plasma with a stably high recovery rate of platelets from a blood sample, platelet-rich plasma produced by the method, and the like.

## Claims

1. A method for producing platelet-rich plasma, comprising:
a step A of subjecting a blood sample from a mammal to centrifugal separation under centrifugal separation conditions of from 100 to 1000 G for from 1 to 30 minutes, and then collecting a supernatant; and
a step B of subjecting the residual blood sample after the collection of the supernatant in the step A to centrifugal separation under centrifugal separation conditions of from 100 to 1000 G for from 1 to 30 minutes, and then collecting a supernatant to obtain the platelet-rich plasma.

2. The method according to claim 1, wherein the centrifugal separation conditions in the Step A are from 100 to 300 G for from 5 to 15 minutes, and the centrifugal separation conditions in the step B are from 100 to 300 G for from 5 to 15 minutes.

3. The method according to claim 1, wherein the blood sample from the mammal to be subjected to the centrifugal separation in the step A is a blood sample stored at from 0 to 35°C for from 1 to 120 hours after the collection from the mammal.

4. The method according to claim 1, wherein the blood sample from the mammal to be subjected to the centrifugal separation in the step A is a blood sample to which an anticoagulant agent has been added after the collection from the mammal.

5. Platelet-rich plasma produced by the method according to any one of claims 1 to 4.
